# EUROPEAN PATENT APPLICATION

(11) **EP 1 584 343 A1**
(43) Date of publication of application: **12.10.2005**
(21) Application number: 05102685.4
(22) Date of filing: 05.04.2005
(51) Int. Cl.: A61M 5/44, A61J 1/16

(54) **Method for packaging thermal reactors, particularly for conditioning containers of fluids for parenteral administration, and associated reactors**

(30) Priority: 08.04.2004 IT MO20040075
(71) Applicant: Sidam di Azzolini Graziano E C. S.a.s., (Prov.of Modena) (IT)
(72) Inventor: AZZOLINI, Graziano, 41032, CAVEZZO MO (IT); ERCOLANI, Simone Pietro, 06125, PERUGIA (IT); CONTI, Paolo, 56123, PISA (IT)
(74) Representative: Modiano, Guido

(57) **Abstract**

A method for packaging thermal reactors, particularly for conditioning containers of fluids for parenteral administration and of the type that comprises an enclosure (2) divided into at least one first compartment (3) and one second compartment (4), mutually separated by a tearable membrane (5) and provided with at least one filling inlet (3a, 4a), the method comprising: introducing a first component (C1) in the first compartment (3) through the respective inlet (3a); introducing a second component (C2), suitable to react with the first component (C1) with an exothermic or endothermic reaction, in the second compartment (4) through the respective inlet (4a); extracting from at least one of the first and second compartments (3, 4) air contained therein through the respective inlet; sealing the inlet (3a) of the first compartment (3), containing the first component (C1); and sealing the inlet (4a) of the second compartment (4), containing the second component (C2). The thermal reactor, particularly for conditioning containers of fluids for parenteral administration, comprises a closed enclosure (2) divided into at least one first compartment (3), which contains a first component (C1), and a second compartment (4), which contains a second component (C2) suitable to react with the first component (C1) with an exothermic or endothermic reaction and is separated from the first compartment (3) by a tearable membrane (5), at least one of the first and second compartments (3, 4) containing respectively the first component (C1) and the second component (C2) substantially in vacuum.

## Description

The present invention relates to a method for packaging thermal reactors, particularly for conditioning containers of fluids for parenteral administration, and to the associated reactors.

It is known that a person affected by a sudden illness, an accident or an injury is often in a state of shock characterized by hypovolemia (blood volume reduction) and by hypothermia (drop in body temperature).

One of the first treatments administered to such a person in first-aid actions performed directly on the site of the illness, accident or injury or during the transport of said person to a health-care facility, consists of the parenteral administration of fluids, such as for example physiological solutions, drugs or other fluids, such as to allow the person to return to normovolemia and normothermia.

These fluids are packaged in containers, such as for example bottles or bags, which have a dispensing outlet with which the input end of a discharge line is associated, the output end of the line being associated with an element for injection into the person being aided.

Often, particularly in procedures performed in open-air locations, the fluids to be administered are at an average temperature that is lower than the normal average body temperature (on the order of 35°-37° C).

To prevent the thermal conditions of the aided person from deteriorating instead of improving, it is therefore necessary to heat the fluids to a preset temperature (comprised between a minimum value on the order of 33° C and a maximum value on the order of 40° C) and keep them thereat, respectively before and during their administration.

Thermal reactors are currently known which are used particularly for the thermal conditioning, particularly the heating, of fluid containers for parenteral administration, which are substantially constituted by an enclosure divided into at least two compartments that are mutually separated by a tearable membrane, one of said compartments containing a first component, the other compartment containing a second component, said components being suitable to react with each other with an exothermic reaction.

The reaction is usually a reaction of solution of the first component (solute) in the second component (solvent).

The first component is generally in the form of solid particles, granules or the like, and is constituted for example by calcium chloride or the like; the second component is generally in the form of a liquid and is constituted for example by water or the like.

At the time of use, an operator acts manually on the enclosure, for example with a compression or squeezing action, applying thereto a pressure that breaks the tearable membrane and thus allows the second component (solvent) to pour from the second compartment into the first compartment, where it reacts with the first component (solute), dissolving it; if the reaction is exothermic, heat is generated.

After activating the reaction, the enclosure is placed in contact with the container of the fluid to be administered in order to condition it thermally and in particular to heat it to the intended temperature.

There are also cases, such as for example a cardiac arrest, in which it is necessary to cool a fluid as neuroprotection; in this case, the reaction between the first component and the second component is endothermic, i.e., it absorbs heat from the outside.

These known types of thermal reactor are not free from drawbacks, including the fact that the reaction between the components that they contain can be activated unintentionally and at inappropriate times by compressions, impacts, or accidental squeezing to which they can be subjected during their storage, movement and handling, which accordingly require particular care and attention.

Therefore, known reactors may lose prematurely their capacity to generate or absorb heat, losing their function and becoming unusable when they are actually needed.

Another drawback of known reactors is that they contain air, which by having a low thermal conductivity acts as an insulator and therefore reduces the exchange of heat between the reactors and the environment outside them and in particular between the reactors and the containers of the fluids to be conditioned.

Moreover, the presence of air in known reactors causes another drawback: it in fact prevents the second component, the one in the liquid state, from diffusing and being distributed throughout the entire volume of the first compartment that contains the first component, the one in the solid state in the form of particles or granules; the reaction surface is thus reduced, and the reaction itself remains incomplete, with a consequent decrease in the efficiency of the reactor.

The aim of the present invention is to eliminate the drawbacks noted above of known thermal reactors, by providing a method for packaging thermal reactors, particularly for conditioning containers of fluids for parenteral administration, that allows to improve the distribution and diffusion, one within the other, of the reacting components and to increase their reaction surface.

An object of the present invention is to obtain thermal reactors that have an improved efficiency and an increased capacity to exchange heat with the environment that lies outside them.

Another object of the present invention is to obtain thermal reactors that have improved functionality and safety, can be activated only intentionally, and do not require particular precautions for their storage, movement and handling.

Within this aim, an object of the present invention is to provide a structure that is simple, relatively easy to provide in practice, safe in use, effective in operation, and has a relatively low cost.

This aim and these and other objects that will become better apparent hereinafter are achieved by the present method for packaging thermal reactors, particularly for conditioning containers of fluids for parenteral administration and of the type that comprises an enclosure divided into at least one first compartment and one second compartment, which are mutually separated by a tearable membrane and are provided with at least one filling inlet, characterized in that it comprises:
- introducing a first component in said first compartment through said respective inlet,
- introducing a second component, suitable to react with said first component with an exothermic or endothermic reaction, in said second compartment through said respective inlet,
- extracting from at least one of said first and second compartments the air contained therein through the respective inlet,
- sealing the inlet of the first compartment, which contains said first component,
- sealing the inlet of the second compartment, which contains said second component.

This aim and these objects are also achieved by the present thermal reactor, particularly for conditioning containers of fluids for parenteral administration, which comprises a closed enclosure divided into at least one first compartment, which contains a first component, and a second compartment, which contains a second component suitable to react with the first component with an exothermic or endothermic reaction and is separated from said first compartment by a tearable membrane, characterized in that at least one of said first and second compartments contains respectively the first component and the second component substantially in vacuum.

Further characteristics and advantages of the present invention will become better apparent from the following detailed description of a preferred but not exclusive embodiment of a method for packaging thermal reactors, particularly for conditioning containers of fluids for parenteral administration, and of the associated reactors, illustrated by way of nonlimiting example in the accompanying drawings, wherein:

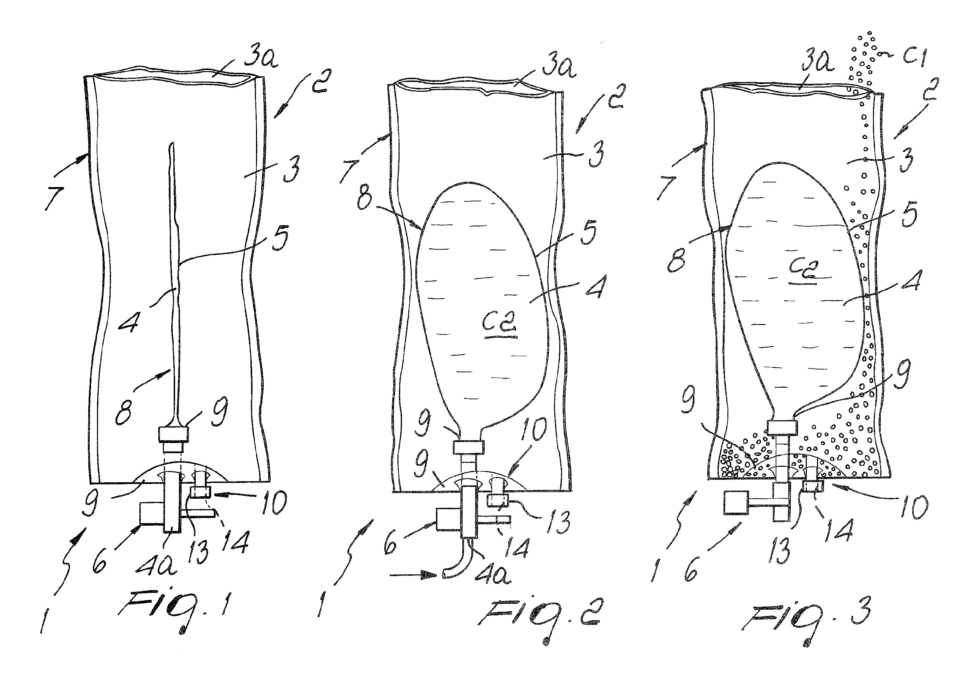

Figure 1 is a schematic view of a reactor according to the invention before packaging;
Figure 2 is a schematic view of the step of the method according to the invention in which the second component is introduced in the second compartment;
Figure 3 is a schematic view of the step of the method according to the invention in which the first component is introduced in the first compartment;
Figure 4 is a schematic view of a thermal reactor according to the invention;
Figure 5 is a schematic view of the activation of a thermal reactor according to the invention;
Figure 6 is a schematic axonometric view of a first aid kit according to the invention;
Figure 7 is a schematic plan view of an element of the kit of Figure 6.

With reference to the figures, the reference numeral 1 generally designates a thermal reactor particularly for conditioning, and more particularly for heating, containers of fluids for parenteral administration, which can be used for example in first-aid actions.

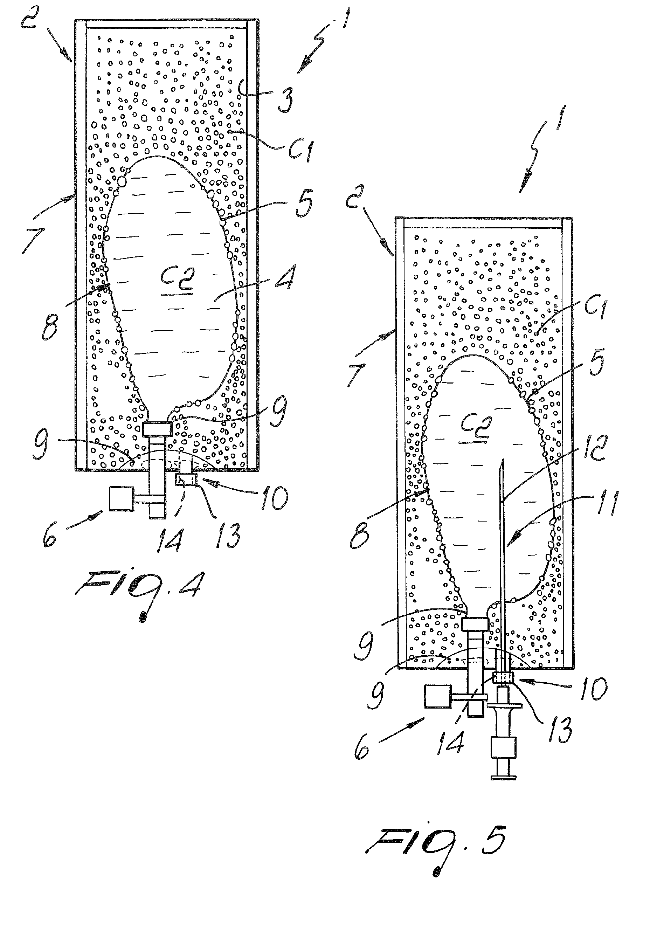

The reactor 1 (Figure 4) comprises an enclosure 2, which is closed and divided into a first compartment 3, which contains a first component C1, and into a second compartment 4, which contains a second component C2, which is suitable to react with the first component C1 with an exothermic or endothermic reaction and is separated from the first compartment 3 by a tearable membrane 5.

If the reaction is exothermic, it generates heat, which is transmitted by the reactor 1 to the environment that lies outside it; if the reaction is endothermic, it absorbs heat from the environment that lies outside the reactor 1.

It should be noted that in the present description, the distinction between the first compartment and the second compartment and between the first component and the second component is merely an example and does not limit the protective scope of the present invention; moreover, alternative solutions, which reproduce the inventive concept of the present invention and in which the enclosure 2 is divided into more than two compartments that contain the same component or different components, are not excluded.

At least one of the first and second compartments 3 and 4 contains respectively the first component C1 and the second component C2 substantially in vacuum; in the embodiment shown in the figures, it is the first component C1 that is packaged substantially in vacuum in the first compartment 3.

The first component C1 is in the form of a loose solid, such as particles, granules or the like, or in liquid form; the second component C2 is in liquid form.

In the case of an exothermic reaction, the first component C1 can be constituted for example by calcium chloride, while the second component C2 can be constituted by water, the solution of the former in the latter being exothermic; however, alternative embodiments of the reactor 1 in which the first and second components C1 and C2 have a different chemical nature are not excluded.

The first compartment 3 comprises a filling inlet 3a, through which the first component C1 is introduced therein and which is for example heat-sealed in the packaged reactor 1.

Likewise, the second compartment comprises a filling inlet 4a, through which the second component C2 is introduced therein and which is sealed for example by means of fastening and sealing means 6 in the packaged reactor 1.

The enclosure 2 is constituted by a first bag 7 or the like, the interior of which forms the first compartment 3, and by a second bag 8, which is shaped like a balloon, bladder or the like, the walls of which are at least partially constituted by the membrane 5; said second bag is accommodated inside the first bag 7 and its interior forms the second compartment 4.

The enclosure 2, i.e., the first and second bags 7 and 8, are made of a material of the type that is flexible, impermeable and resistant to the chemical attack of the first and second components C1 and C2, such as for example silicone; the first bag 7 further comprises at least one heat-conducting portion, through which it exchanges heat with the environment that lies outside it.

The first bag 7 and the second bag 8 are mutually associated at portions 9 of their respective walls at which insertion means 10 are provided for inserting a tool 11 for tearing the membrane 5, such as for example a needle 12.

The insertion means 10 are constituted by an inlet 13, which is provided with retention and sealing means, such as an insert 14 made of elastic/elastomeric material, which is accommodated therein; the insert 14 is suitable to be crossed by the tool 11 and to close, once the tool 11 has been extracted, the opening formed therein by said tool, preventing the escape of the first and second components C1 and C2 from the enclosure 2 and the inflow of external air therein.

The reactor 1 can be used for the thermal conditioning of containers of fluids for parenteral administration.

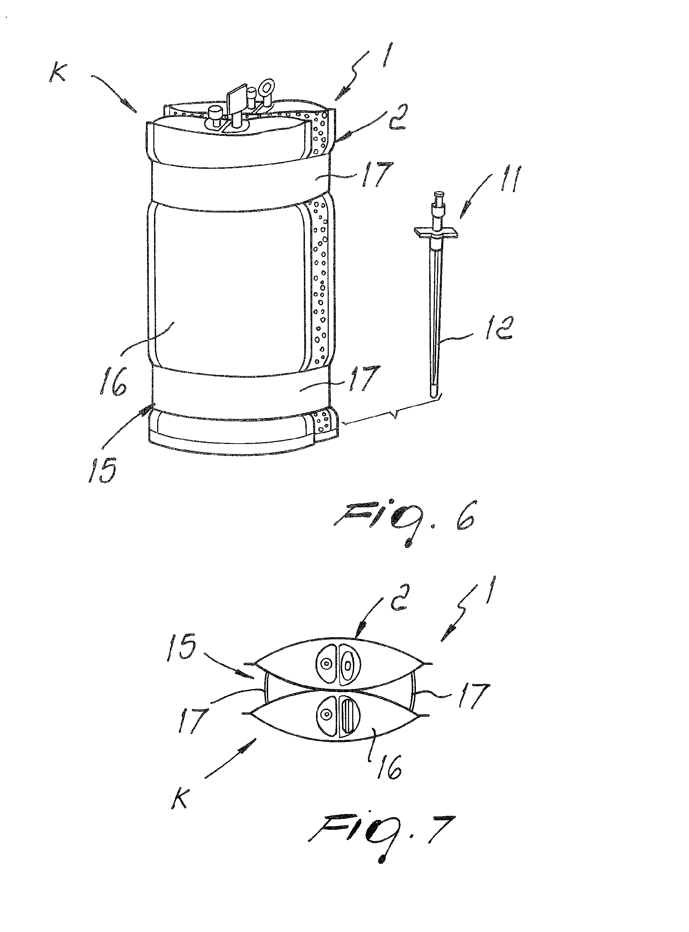

Figures 6 and 7 illustrate a kit K for first-aid actions, which comprises a reactor 1 coupled by anchoring means 15 to a container of a fluid for parenteral administration, such as a pouch 16. The kit K can further comprise the tool 11 for tearing the membrane 5, such as the needle 12.

The enclosure 2 overlaps the pouch 16 at its thermally-conducting portion; the anchoring means 15 are of the type of adhesive tapes 17, straps or the like.

With particular reference to the reactor 1 described and illustrated above, the method for packaging it comprises the steps of:
- introducing the second component C2 in the second compartment 4 (i.e., in the second bag 8) through the respective open inlet 4a, until said second compartment is filled,
- sealing the inlet 4a with the fastening and sealing means 6, such as for example a clamp and a pad, or such as a heat seal,
- introducing the first component C1 in the first compartment 3 (i.e., in the first bag 7) through the respective open inlet 3 a,
- extracting from the first compartment 3, which contains the first component C1, the air contained therein through the respective inlet 3a until a condition of substantial vacuum is reached,
- sealing the inlet 3a, for example by heat-sealing its lips, of the first compartment 3 that contains the first component C1 packaged therein substantially in vacuum.

The method according to the invention further comprises associating with the enclosure 2, before filling it with the first and second components C1 and C2, i.e., during the preparation of the first and second bags 7 and 8, the means 10 for inserting the tool 11 for tearing the membrane 5.

The operation of the invention can be deduced easily by the person skilled in the art.

In practice it has been found that the described invention achieves the intended aim and objects.

The reactor according to the invention has improved efficiency and increased heat transmission capacity; since it is in fact substantially devoid of air inside it, it allows the second and first components to diffuse and distribute better within each other and to increase their reaction surface.

The reactor according to the invention, by virtue of the absence of air and the need to act with an appropriate tearing tool, is further safer, can be activated only intentionally, and does not require particular care in its storage, handling and movement.

Finally, the reactor according to the invention, packaged in a kit, already coupled to a container of the fluid to be administered parenterally, facilitates and speeds up the maneuvers that health operators must perform during first-aid actions.

The reactor has in fact the appearance of a cartridge that is ready for use and can be replaced with another backup cartridge without requiring the operators to handle separately the depleted reactor and fluid container and the backup ones, with an obvious saving in time and increase in efficiency of the action.

The invention thus conceived is susceptible of numerous modifications and variations, all of which are within the scope of the appended claims.

All the details may further be replaced with other technically equivalent ones.

In practice, the materials used, as well as the shapes and the dimensions, may be any according to requirements without thereby abandoning the scope of the protection of the appended claims.

The disclosures in Italian Patent Application No. MO2004A000075 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A method for packaging thermal reactors, particularly for conditioning containers of fluids for parenteral administration and of the type that comprises an enclosure (2) divided into at least one first compartment (3) and one second compartment (4), which are mutually separated by a tearable membrane (5) and are provided with at least one filling inlet (3a, 4a), **characterized in that** it comprises the steps of:
- introducing a first component (C1) in said first compartment (3) through said respective inlet (3 a),
- introducing a second component (C2), suitable to react with said first component (C1) with an exothermic or endothermic reaction, in said second compartment (4) through said respective inlet (4a),
- extracting from at least one of said first and second compartments (3, 4) the air contained therein through the respective inlet (3 a, 4a),
- sealing the inlet (3a) of the first compartment (3), which contains said first component (C1),
- sealing the inlet (4a) of the second compartment (4), which contains said second component (C2).

2. The method according to claim 1, **characterized in that** it comprises associating with said enclosure (2) means (10) for inserting a tool (11) for tearing said membrane (5).

3. The method according to one or more of the preceding claims, comprising the step of providing said insertion means (10) with retention and sealing means (4) suitable to prevent the escape of said first and second components and the inflow of the air that lies outside said enclosure.

4. The method according to one or more of the preceding claims, comprising the steps of providing said insertion means (10) with an inlet (13) and said retention and sealing means with an insert (14) made of elastic/elastomeric material, which is accommodated in said inlet (13) and is suitable to be crossed by said tool (11) and to close the opening formed therein by said tool (11).

5. The method according to one or more of the preceding claims, comprising the step of providing at least one of said first and second components (C1, C2) in a loose solid form, such as particles, granules or the like, the other component being provided in the form of a fluid.

6. The method according to one or more of the preceding claims, **characterized in that** said introduction step consists in filling said first compartment (3) or said second compartment (4).

7. The method according to one or more of the preceding claims, **characterized in that** said extraction step follows said introduction.

8. The method according to one or more of the preceding claims, **characterized in that** said sealing step occurs after said extraction or said introduction step.

9. The method according to one or more of the preceding claims, **characterized in that** said sealing step comprises heat-sealing the lips of said inlet (3a).

10. The method according to one or more of the preceding claims, **characterized in that** said sealing step comprises applying fastening means (6) to said inlet (4a).

11. The method according to one or more of the preceding claims, comprising the step of providing said enclosure (2) with a first bag (7) or the like, the interior of which forms said first compartment (3), and a second bag or the like, the walls of which are at least partially constituted by said membrane (5), said second bag (8) being accommodated in said first bag (7) and its inside forming said second compartment (4).

12. The method according to one or more of the preceding claims, wherein said enclosure (2) is made of a material of the flexible type.

13. The method according to one or more of the preceding claims, comprising providing said enclosure (2) with at least one heat-conducting portion.

14. The method according to one or more of the preceding claims, comprising providing said first and second bags (7, 8) mutually associated at at least one portion (9) of their respective walls.

15. The method according to one or more of the preceding claims, comprising associating said insertion means (10) at said portion (9).

16. A thermal reactor, particularly for conditioning containers of fluids for parenteral administration, comprising a closed enclosure (2), which is divided into at least one first compartment (3), which contains a first component (C1), and into a second compartment (4), which contains a second component (C2) suitable to react with the first component (C1) with an exothermic or endothermic reaction and is separated from said first compartment (3) by a tearable membrane (5), **characterized in that** at least one of said first and second compartments (3, 4) contains respectively the first and second components (C1, C2) substantially in vacuum.

17. The reactor according to claim 16, **characterized in that** said enclosure (2) comprises means (10) for inserting a tool (11) for tearing said membrane (5).

18. The reactor according to one or more of claims 16 to 17, **characterized in that** said insertion means (10) comprise retention and sealing means (14), which are suitable to prevent the escape of said first and second components (C1, C2) and the inflow of the air that lies outside said enclosure (2).

19. The reactor according to one or more of claims 16 to 18, **characterized in that** said insertion means (10) comprise an inlet (13) and **in that** said retention and sealing means comprise an insert (14) made of elastic/elastomeric material, which is accommodated in said inlet (13) and is suitable to be crossed by said tool (11) and to close the opening formed therein by said tool.

20. The reactor according to one or more of claims 16 to 19, **characterized in that** at least one of said first and second components (C1, C2) is in the form of a loose solid, such as particles, granules or the like, the other component being in fluid form.

21. The reactor according to one or more of claims 16 to 20, **characterized in that** said enclosure (2) comprises a first bag (7) or the like, the interior of which forms said first compartment (3), and a second bag (8) or the like, the walls of which are at least partially constituted by said membrane (5), said second bag (8) being accommodated in said first bag (7) and its interior forming said second compartment (4).

22. The reactor according to one or more of claims 16 to 21, **characterized in that** said enclosure (2) is made of a material of the flexible type.

23. The reactor according to one or more of claims 16 to 22, **characterized in that** said enclosure (2) comprises at least one heat-conducting portion.

24. The reactor according to one or more of claims 16 to 23, **characterized in that** said first and second bags (7, 8) are mutually associated at at least one portion (9) of their respective walls.

25. The reactor according to one or more of claims 16 to 24, **characterized in that** said insertion means (10) are associated at said portion (9).

26. The use of a thermal reactor (1) according to one or more of claims 16 to 25 for the thermal conditioning of containers of fluids for parenteral administration.

27. A first-aid kit, **characterized in that** it comprises a thermal reactor (1) according to one or more of claims 16 to 25, which is coupled by way of anchoring means (15) to a container (16) of a fluid for parenteral administration.

28. The kit according to claim 27, **characterized in that** said enclosure (2) overlaps said container (16) at least at said heat-conducting portion thereof.

29. The kit according to one or more of claims 27 and 28, **characterized in that** said anchoring means (15) are of the type of straps, adhesive tapes (17) or the like.

30. The kit according to one or more of claims 27 to 29, **characterized in that** said container is constituted by a pouch (16).

31. The kit according to one or more of claims 27 to 30, **characterized in that** it comprises a tool (11) for tearing said membrane (5).

32. The kit according to one or more of claims 27 to 31, **characterized in that** said tearing tool (11) is of the type of a needle or the like.
